# EUROPEAN PATENT APPLICATION

(11) **EP 4 397 317 A2**
(43) Date of publication of application: **10.07.2024**
(21) Application number: 24170976.5
(22) Date of filing: 30.08.2018
(51) Int. Cl.: A61K 39/395

(54) **COMBINATION THERAPY OF LASMIDITAN AND A CGRP ANTAGONIST FOR USE IN THE TREATMENT OF MIGRAINE**

(30) Priority: 06.09.2017 US 201762554726 P
(62) Divisional of application: 18766557.5
(71) Applicant: Eli Lilly and Company, Indianapolis, IN 46285 (US)
(72) Inventor: AURORA, Sheena, Indianapolis, 46206-6288 (US); JOHNSON, Kirk Willis, Indianapolis, 46206-6288 (US); KREGE, John Henry, Indianapolis, 46206-6288 (US)
(74) Representative: Faggiani, Davide

(57) **Abstract**

The present invention relates to combinations of lasmiditan and a calcitonin gene-related peptide (CGRP) antagonist such as rimegepant, ubrogepant, olcegepant, and MK-8031 and to methods of using the combinations for treatment of migraine, particularly migraine inadequately controlled by lasmiditan or a CGRP antagonist therapy alone, and more particularly, to treat therapy resistant migraine which is defined herein as migraine refractory to two or more prior monotherapy and/or dual therapy treatment regimens.

## Description

The present invention relates to combinations of lasmiditan and a calcitonin gene-related peptide (CGRP) antagonist, for example the combination of galcanezumab and lasmiditan, and to methods of using the combinations for treatment of migraine, particularly migraine inadequately controlled by lasmiditan or a CGRP antagonist therapy alone, and more particularly, to treat therapy resistant migraine which is defined herein as migraine refractory to two or more prior monotherapy and/or dual therapy treatment regimens.

The primary headache disorders, including migraine, are among the most common diseases and leading causes of disability worldwide. Migraine affects over 14% of adults worldwide. The available treatment options for migraine have unsatisfactory rates of efficacy, tolerability and patient adherence. In the 2013 Global Burden of Disease Study, migraine accounted for over half of all years lost to disability that were attributed to neurological disorders (New strategies for the treatment and prevention of primary headache disorders, N. M. Schuster & A. M. Rapoport, Nature Reviews Neurology (2016) 12, 635-650). Migraine is typically characterized by attacks of 1-3 days of severe headache, associated with nausea, vomiting, photo- and phonophobia (migraine without aura), and, in one third of patients, neurological aura symptoms (migraine with aura) (Goadsby, P.J. et al., New England Journal of Medicine 2002; 346: 257-270).

Lasmiditan, 2,4,6-trifluoro-N-[6-(1-methyl-piperidin-4-ylcarbonyl)-pyridin-2-yl]-benzamide (Compound I) is a selective and highly potent 5-HT-1F receptor agonist which is in development for treatment of migraine (See eg. Lasmiditan for the Treatment of Migraine, Capi, M. et al., Expert Opinion Investigational Drugs, (2017), Vol. 26, NO. 2, 227-234).

Calcitonin gene-related peptide (CGRP) is a 37-amino acid peptide found primarily in the C and Ad sensory fibers arising from the dorsal root and trigeminal ganglia, as well as the central nervous system. CGRP is a pain-signalling neuropeptide and potent vasodilator that is released from trigeminal sensory afferents and the spinal trigeminal nucleus. The role of CGRP in headache and migraine has been established in the art and a number of clinical studies are currently evaluating the use of anti-CGRP antibodies for the treatment of headaches and migraine (See, for example, Dodick et al. Lancet Neurolology; 13(9): 885-892 (2014)).

The present invention relates to combinations of lasmiditan and a calcitonin gene-related peptide (CGRP) antagonist, for example the combination of lasmiditan and galcanezumab, and to methods of using the combinations to treat migraine. More particularly, the present invention relates to the use of combinations of lasmiditan and a calcitonin gene-related peptide (CGRP) antagonist for the treatment of migraine inadequately controlled by lasmiditan or a CGRP antagonist therapy alone. More particularly, the present invention relates to the use of combinations of lasmiditan and a calcitonin gene-related peptide (CGRP) antagonist for the treatment of therapy resistant migraine which is defined herein as migraine refractory to two or more prior monotherapy and/or dual therapy treatment regimens.

The management of patients with migraine is often unsatisfactory because available acute and preventive therapies are either ineffective or poorly tolerated. The acute treatment of migraine attacks has been limited to the use of analgesics, combinations of analgesics with caffeine, ergotamines, and the triptans. (For a description of such agents see e.g. New Therapeutic Approaches for the Prevention and Treatment of Migraine, Diener, H.C. et al., (2015) Lancet Neurololgy, 14:1010-22). Lasmiditan represents an innovative approach for acute migraine therapy by selectively targeting 5-HT-1F. While many patients will be able to successfully manage migraine episodes by treatment with lasmiditan or galcanezumab alone, a population of patients will fail to successfully manage their migraine attacks with either of these agents individually. These patients may have a number of migraine day's per-month that continues to be significantly disabling. Further, some patients, referred to herein as therapy resistant migraine patients, will fail to successfully manage their migraine attacks and will suffer from migraines which are refractory to two or more prior monotherapy and/or dual therapy treatment regimens. As defined herein, therapy resistant migraine patients will be those that continue to suffer from 3 or more migraine days per month despite two or more prior monotherapy and/or dual therapy treatment regimens. As used herein, two or more prior monotherapy and/or dual therapy treatment regimens means prior unsatisfactory treatment attempts with a monotherapy or dual therapy regimen, such as triptans, ergotamines, nonsteroidal anti-inflammatory drugs (NSAIDs), nonnarcotic analgesics, and caffeine, either alone or two such agents in combination. Therapy resistant patients have yet to achieve substantial freedom from recurrent migraine, and thus represent a critical unmet need. Failure of these therapy resistant migraine patients to achieve adequate relief from multiple prior treatment regimens demonstrates that their disease is particualry difficult to treat, and efficacy in this population represents a surprising and superior outcome.

Lasmiditan (COL 144, LY 573144, CAS Registry No. 439239-90-4) as used in the combinations of the present invention can be described chemically as 2,4,6-trifluoro-N-[6-(1-methyl-piperidin-4-ylcarbonyl)-pyridin-2-yl]-benzamide and can be structurally represented as Compound I: 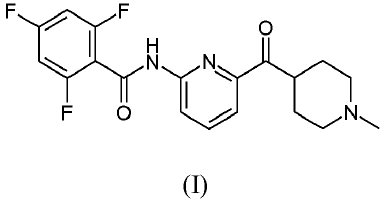 As used herein, Compound I includes pharmaceutically acceptable salts thereof, including but not limited to 2,4,6-Trifluoro-N-[6-(1-methyl-piperidin-4-ylcarbonyl)-pyridin-2-yl]-benzamide mono-hydrochloride salt, and 2,4,6-Trifluoro-N-[6-(1-methyl-piperidine-4-carbonyl)-pyridin-2-yl]-benzamide hemi-succinate salt. Methods of preparing lasmiditan and salts and certain formulations and dosage forms thereof are known to the skilled artisan, and are described in WO 03/084949 and WO 2011/123654.

Galcanezumab (LY 2951742, CAS Registry No. 1578199-75-3) as used in the combinations of the present invention can be described as a monoclonal antibody targeting calcitonin gene-related peptide (CGRP). Galcanezumab monotherapy is being developed for migraine and cluster headache (See e.g. New players in the preventive treatment of migraine, Mitsikostas, Dimos D.; Rapoport, Alan M., BMC Medicine (2015), 13, 279/1-279/7, and Translational pharmacodynamics of calcitonin gene-related peptide monoclonal antibody LY2951742 in a capsaicin-induced dermal bloodflow model, Vermeersch, S., et al. Journal of Pharmacology and Experimental Therapeutics (2015), 354(3), 350-357). Methods of preparing galcanezumab are known to the skilled artisan and described in WO 2011/156324. Other CGRP antagonists useful in the combinations of the present invention and known to the skilled artisan include eptinesumab (ALD403), fremanezumab (TEV-48125), erenumab (AMG334), ubrogepant (MK-1602), MK-8031, olcegepant, or rimegepant (BHV-3000; BMS-927711) (See e.g. New strategies for the treatment and prevention of primary headache disorders, N. M. Schuster & A. M. Rapoport, Nature Reviews Neurology (2016) 12, 635-650). CGRP antagonists useful in the combinations of the present invention and known to the skilled artisan include small molecule antagonists and monoclonal antibody antagonists targeting CGRP itself or its receptors. Methods of preparing other CGRP antagonists are known to the skilled artisan.

There exists a need for more and different therapies that may prove to be effective in treating migraine, in particular for the treatment of migraine inadequately controlled by lasmiditan or a CGRP antagonist therapy alone, and there remains a critical need for treatment of therapy resistant migraine which is defined herein as migraine refractory to two or more prior monotherapy and/or dual therapy treatment regimens.

Novel methods are provided herein for the use of combinations of lasmiditan and a calcitonin gene-related peptide (CGRP) antagonist, for example the combination of lasmiditan and galcanezumab, to treat migraine and therapy resistant migraine. It is believed that the combination of lasmiditan and galcanezumab for the treatment of migraine will be superior to either monotherapy alone, by combined action on the CGRP pathway in combination with a complimentary action of lasmiditan to decrease glutamate signaling. It is believed the combination of these pharmacological properties will result in superior efficacy for migraine treatment in patients who suffer from therapy resistant migraines.

Accordingly, the present invention provides lasmiditan for use in simultaneous, separate, or sequential combination with a calcitonin gene-related peptide (CGRP) antagonist, for example galcanezumab, in the treatment of migraine, in particular for the treatment of migraine inadequately controlled by lasmiditan or a CGRP antagonist therapy alone, and for treatment of therapy resistant migraine in a patient. More particularly, the migraine patient treated is one who suffers from migraines that are inadequately controlled by lasmiditan or a CGRP antagonist therapy alone. More particularly, the migraine patient treated is one who suffers from therapy resistant migraines, which are defined herein as migraine refractory to two or more prior monotherapy and/or dual therapy treatment regimens.

Combinations of lasmiditan and a calcitonin gene-related peptide (CGRP) antagonist, for example the combination of lasmiditan and galcanezumab, and methods of using the combinations to treat migraine, particularly migraine inadequately controlled by lasmiditan or a CGRP antagonist therapy alone, and more particularly, to treat therapy resistant migraine, employ certain doses and dosing regimens of lasmiditan and galcanezumab, which are described below.

The present invention relates to the combination use of pharmaceutical compositions comprising an amount of lasmiditan or a pharmaceutically acceptable salt thereof and a pharmaceutically acceptable diluent or carrier, wherein for oral administration said composition comprises 50-400 mg per dose of lasmiditan or a pharmaceutically acceptable salt thereof, and for buccal, sublingual, nasal/intranasal, transdermal, subcutaneous, injectable, intravenous or intramuscular administration, said composition comprises up to 200 mg per dose of lasmiditan, or a pharmaceutically acceptable salt thereof, further wherein said composition is administered one, two, or three times daily. The present invention relates to the combination use of a pharmaceutical composition of lasmiditan, wherein said composition is for oral administration and the amount of lasmiditan or pharmaceutically acceptable salt thereof is from 50 mg to 400 mg per dose. The present invention relates to the combination use of a pharmaceutical composition of lasmiditan, wherein the amount of lasmiditan is 50 mg per dose. The present invention relates to the combination use of a pharmaceutical composition of lasmiditan, wherein the amount of lasmiditan is 100 mg per dose. The present invention relates to the combination use of a pharmaceutical composition of lasmiditan, wherein the amount of lasmiditan is 200 mg per dose. The present invention relates to the combination use of a pharmaceutical composition of lasmiditan, wherein the amount of lasmiditan is 400 mg per dose. The present invention relates to the combination use of a pharmaceutical composition of lasmiditan, wherein said composition is for buccal, sublingual, nasal/intranasal, transdermal, subcutaneous, injectable, intravenous, or intramuscular administration and the amount of lasmiditan or pharmaceutically acceptable salt thereof administered is up to 200 mg per dose. The present invention relates to the combination use of a pharmaceutical composition of lasmiditan, wherein the amount of lasmiditan or a pharmaceutically acceptable salt thereof administered is 20 mg to 200 mg per dose. The present invention relates to the combination use of a pharmaceutical composition of lasmiditan, wherein the amount of lasmiditan or a pharmaceutically acceptable salt thereof administered is from 20 to 60 mg per dose. The present invention relates to the combination use of a pharmaceutical composition of lasmiditan, wherein the amount of lasmiditan or a pharmaceutically acceptable salt thereof administered is from 20 to 30 mg per dose. The present invention relates to the combination use of a pharmaceutical composition of lasmiditan, wherein the administration is intravenous and the amount of lasmiditan or a pharmaceutically acceptable salt thereof administered is up to 200 mg per dose. The present invention relates to the combination use of a pharmaceutical composition of lasmiditan, wherein the administration of lasmiditan or a pharmaceutically acceptable salt thereof is intravenous over a period of about 20 minutes.

The present invention relates to the combination use of a pharmaceutical composition of lasmiditan, wherein the composition comprises the hemi-succinate salt of lasmiditan. The present invention relates to the combination use of a pharmaceutical composition of lasmiditan, wherein the composition comprises the hemi-succinate salt of lasmiditan and the amount administered is 50 mg per dose. The present invention relates to the combination use of a pharmaceutical composition of lasmiditan, wherein the composition comprises the hemi-succinate salt of lasmiditan and the amount administered is 100 mg per dose. The present invention relates to the combination use of a pharmaceutical composition of lasmiditan, wherein the composition comprises the hemi-succinate salt of lasmiditan and the amount administered is 200 mg per dose.

The present invention relates to the combination use of a pharmaceutical composition of lasmiditan, wherein the dose of lasmiditan or a pharmaceutically acceptable salt thereof is administered one time daily. The present invention relates to the combination use of a pharmaceutical composition of lasmiditan, wherein the dose of lasmiditan or a pharmaceutically acceptable salt thereof is administered two times daily. The present invention relates to the combination use of a pharmaceutical composition of lasmiditan, wherein the dose of lasmiditan or a pharmaceutically acceptable salt thereof is administered three times daily.

The present invention relates to a method for the treatment of migraine, in a patient in need thereof, comprising orally administering to the patient 50-400 mg per dose of lasmiditan or a pharmaceutically acceptable salt thereof and a pharmaceutically acceptable diluent or carrier. The present invention relates to a method for the treatment of migraine, in a patient in need thereof, comprising orally administering to the patient 50-400 mg per dose of lasmiditan or a pharmaceutically acceptable salt thereof and a pharmaceutically acceptable diluent or carrier, wherein said composition is administered one, two, or three times daily.

The present invention relates to a method for the treatment of migraine, in a patient in need thereof, comprising orally administering to the patient 50 mg per dose of lasmiditan, or a pharmaceutically acceptable salt thereof, and a pharmaceutically acceptable diluent or carrier. The present invention relates to a method for the treatment of migraine, in a patient in need thereof, comprising orally administering to the patient 50 mg per dose of lasmiditan, or a pharmaceutically acceptable salt thereof, and a pharmaceutically acceptable diluent or carrier, wherein said composition is administered one or two times daily.

The present invention relates to a method for the treatment of migraine, in a patient in need thereof, comprising orally administering to the patient 100 mg per dose of lasmiditan or a pharmaceutically acceptable salt thereof, and a pharmaceutically acceptable diluent or carrier. The present invention relates to a method for the treatment of migraine, in a patient in need thereof, comprising orally administering to the patient 100 mg per dose of lasmiditan, or a pharmaceutically acceptable salt thereof, and a pharmaceutically acceptable diluent or carrier, wherein said composition is administered one or two times daily.

The present invention relates to a method for the treatment of migraine, in a patient in need thereof, comprising orally administering to the patient 200 mg per dose of lasmiditan or a pharmaceutically acceptable salt thereof, and a pharmaceutically acceptable diluent or carrier. The present invention relates to a method for the treatment of migraine, in a patient in need thereof, comprising orally administering to the patient 200 mg per dose of lasmiditan, or a pharmaceutically acceptable salt thereof, and a pharmaceutically acceptable diluent or carrier, wherein said composition is administered one or two times daily.

The present invention relates to a method for the treatment of migraine, in a patient in need thereof, comprising the buccal, sublingual, nasal/intranasal, transdermal, subcutaneous, injectable, intravenous or intramuscular administration to the patient of 50-400 mg per dose of lasmiditan, or a pharmaceutically acceptable salt thereof, and a pharmaceutically acceptable diluent or carrier.

The present invention relates to a method for the treatment of migraine, in a patient in need thereof, comprising the buccal, sublingual, nasal/intranasal, transdermal, subcutaneous, injectable, intravenous or intramuscular administration to the patient 50-400 mg per dose of lasmiditan, or a pharmaceutically acceptable salt thereof, and a pharmaceutically acceptable diluent or carrier, wherein said composition is administered one, two, or three times daily.

The present invention relates to a method for the combination treatment of migraine, in a patient in need thereof, comprising administering to the patient a dose of 120 mg of galcanezumab. The present invention relates to a method for the combination treatment of migraine, in a patient in need thereof, comprising administering to the patient a dose of 240 mg of galcanezumab. The present invention relates to a method for the combination treatment of migraine, in a patient in need thereof, comprising administering to the patient a dose of 300 mg of galcanezumab. The present invention relates to a method for the combination treatment of migraine, in a patient in need thereof, comprising administering to the patient a dose of 360 mg of galcanezumab. Preferably, the dose of galcanezumab is administered at weekly, semi-monthly, monthly or quarterly intervals. More preferably, the galcanezumab administration is monthly. The present invention relates to a method for the combination treatment of migraine, in a patient in need thereof, comprising administering to the patient an initial loading dose of 240 mg of galcanezumab followed by a monthly maintenance dose of 120 mg of galcanezumab. The present invention relates to a combination method to treat episodic migraine in a patient comprising administering a monthly subcutaneous dose of 120 mg of galcanezumab. As used herein, the combination treatments administering galcanezumab are by the regimens provided above.

Another aspect of the present invention relates to the use of lasmiditan in combination with galcanezumab, as a medicament, and in particular a medicament adapted for the treatment of migraine in humans.

In another embodiment the invention provides a method for use of Lasmiditan in simultaneous, separate, or sequential combination with a calcitonin gene-related peptide (CGRP) antagonist in the treatment of migraine in a patient.

In another embodiment the invention provides a method for use of Lasmiditan in simultaneous, separate, or sequential combination with galcanezumab in the treatment of migraine in a patient.

In another embodiment the invention provides a method for use of Lasmiditan in simultaneous, separate, or sequential combination with a calcitonin gene-related peptide (CGRP) antagonist in the treatment of migraine in a patient inadequately controlled by lasmiditan or a CGRP antagonist therapy alone.

In another embodiment the invention provides a method for use of Lasmiditan in simultaneous, separate, or sequential combination with galcanezumab in the treatment of migraine in a patient inadequately controlled by lasmiditan or galcanezumab therapy alone.

In another embodiment the invention provides a method for use of Lasmiditan in simultaneous, separate, or sequential combination with a calcitonin gene-related peptide (CGRP) antagonist in the treatment of migraine in a patient suffering from therapy resistant migraine wherein the patients migraines have been refractory to two or more prior monotherapy and/or dual therapy treatment regimens.

In another embodiment the invention provides a method for use of Lasmiditan in simultaneous, separate, or sequential combination with galcanezumab in the treatment of migraine in a patient suffering from therapy resistant migraine wherein the patients migraines have been refractory to two or more prior monotherapy and/or dual therapy treatment regimens.

In another embodiment the invention provides a method for use of Lasmiditan in simultaneous, separate, or sequential combination with galcanezumab in the treatment of migraine in a patient, wherein galcanezumab is administered in an initial loading dose of 240 mg of followed by a monthly maintenance dose of 120 mg, and lasmiditan is administered at a dose of 50 mg once or twice a day.

In another embodiment the invention provides a method for use of Lasmiditan in simultaneous, separate, or sequential combination with galcanezumab in the treatment of migraine in a patient, wherein galcanezumab is administered in an initial loading dose of 240 mg of followed by a monthly maintenance dose of 120 mg, and lasmiditan is administered at a dose of 100 mg once or twice a day.

In another embodiment the invention provides a method for use of Lasmiditan in simultaneous, separate, or sequential combination with galcanezumab in the treatment of migraine in a patient, wherein galcanezumab is administered in an initial loading dose of 240 mg of followed by a monthly maintenance dose of 120 mg, and lasmiditan is administered at a dose of 200 mg once or twice a day.

In another embodiment the invention provides a method for use of Lasmiditan in simultaneous, separate, or sequential combination with galcanezumab in the treatment of migraine in a patient, wherein galcanezumab is administered at a monthly dose of 120 mg, and lasmiditan is administered at a dose of 50 mg once or twice a day.

In another embodiment the invention provides a method for use of Lasmiditan in simultaneous, separate, or sequential combination with galcanezumab in the treatment of migraine in a patient, wherein galcanezumab is administered at a monthly dose of 120 mg, and lasmiditan is administered at a dose of 100 mg once or twice a day.

In another embodiment the invention provides a method for use of Lasmiditan in simultaneous, separate, or sequential combination with galcanezumab in the treatment of migraine in a patient, wherein galcanezumab is administered at a monthly dose of 120 mg, and lasmiditan is administered at a dose of 200 mg once or twice a day.

In another embodiment the invention provides a method for use of Lasmiditan in simultaneous, separate, or sequential combination with galcanezumab in the treatment of migraine in a patient, wherein galcanezumab is administered at a monthly dose of 120 mg, and lasmiditan is administered at a dose of 50 mg once a day.

In another embodiment the invention provides a method for use of Lasmiditan in simultaneous, separate, or sequential combination with galcanezumab in the treatment of migraine in a patient, wherein galcanezumab is administered at a monthly dose of 120 mg, and lasmiditan is administered at a dose of 100 mg once a day.

In another embodiment the invention provides a method for use of Lasmiditan in simultaneous, separate, or sequential combination with galcanezumab in the treatment of migraine in a patient, wherein galcanezumab is administered at a monthly dose of 120 mg, and lasmiditan is administered at a dose of 200 mg once a day.

In another embodiment the invention provides a method for use of Lasmiditan in simultaneous, separate, or sequential combination with a calcitonin gene-related peptide (CGRP) antagonist in the treatment of a headache selected from the group consisting of episodic headache, chronic headache, chronic cluster headache, or episodic cluster headache in a patient.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient.

In another embodiment the invention provides a method for use of Lasmiditan in simultaneous, separate, or sequential combination with a calcitonin gene-related peptide (CGRP) antagonist in the treatment of a headache selected from the group consisting of episodic headache, chronic headache, chronic cluster headache, or episodic cluster headache in a patient inadequately controlled by lasmiditan or a CGRP antagonist therapy alone.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient inadequately controlled by lasmiditan or galcanezumab therapy alone.

In another embodiment the invention provides a method for use of Lasmiditan in simultaneous, separate, or sequential combination with a calcitonin gene-related peptide (CGRP) antagonist in the treatment of a headache selected from the group consisting of episodic headache, chronic headache, chronic cluster headache, or episodic cluster headache in a patient suffering from therapy resistant headaches wherein the patients headaches have been refractory to two or more prior monotherapy and/or dual therapy treatment regimens.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient suffering from therapy resistant headaches wherein the patients headaches have been refractory to two or more prior monotherapy and/or dual therapy treatment regimens.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered in an initial loading dose of 240 mg of followed by a monthly maintenance dose of 120 mg, and lasmiditan is administered at a dose of 50 mg once or twice a day.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered in an initial loading dose of 240 mg of followed by a monthly maintenance dose of 120 mg, and lasmiditan is administered at a dose of 100 mg once or twice a day.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered in an initial loading dose of 240 mg of followed by a monthly maintenance dose of 120 mg, and lasmiditan is administered at a dose of 200 mg once or twice a day.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 50 mg once or twice a day.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 100 mg once or twice a day.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 200 mg once or twice a day.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 50 mg once a day.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 100 mg once a day.

In another embodiment the invention provides a method for use of Lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 200 mg once a day.

In another embodiment the invention provides a method of treating migraine in a patient, comprising administering simultaneously, separately, or sequentially to a patient in need of such a treatment, an effective amount of lasmiditan in combination with an effective amount of a calcitonin gene-related peptide (CGRP) antagonist.

In another embodiment the invention provides a method of treating migraine in a patient, comprising administering simultaneously, separately, or sequentially to a patient in need of such a treatment, an effective amount of lasmiditan in combination with an effective amount of galcanezumab.

In another embodiment the invention provides a method of treating migraine in a patient, comprising administering simultaneously, separately, or sequentially to a patient in need of such a treatment, an effective amount of lasmiditan in combination with an effective amount of a calcitonin gene-related peptide (CGRP) antagonist, wherein migraine in the patient was inadequately controlled by lasmiditan or a CGRP antagonist therapy alone.

In another embodiment the invention provides a method of treating migraine in a patient, comprising administering simultaneously, separately, or sequentially to a patient in need of such a treatment, an effective amount of lasmiditan in combination with an effective amount of galcanezumab, wherein migraine in the patient was inadequately controlled by lasmiditan or galcanezumab therapy alone.

In another embodiment the invention provides a method of treating migraine in a patient, comprising administering simultaneously, separately, or sequentially to a patient in need of such a treatment, an effective amount of lasmiditan in combination with an effective amount of a calcitonin gene-related peptide (CGRP) antagonist, wherein the patient suffers from therapy resistant migraine wherein the patients migraines have been refractory to two or more prior monotherapy and/or dual therapy treatment regimens.

In another embodiment the invention provides a method of treating migraine in a patient, comprising administering simultaneously, separately, or sequentially to a patient in need of such a treatment, an effective amount of lasmiditan in combination with an effective amount of galcanezumab, wherein the patient suffers from therapy resistant migraine wherein the patients migraines have been refractory to two or more prior monotherapy and/or dual therapy treatment regimens. As described below, the preceding methods of treatment represent "one of the embodiments above".

In another embodiment the invention provides a method of any one of the embodiments above, wherein galcanezumab is administered in an initial loading dose of 240 mg of followed by a monthly maintenance dose of 120 mg, and lasmiditan is administered at a dose of 50 mg once or twice a day.

In another embodiment the invention provides a method of any one of the embodiments above, wherein galcanezumab is administered in an initial loading dose of 240 mg of followed by a monthly maintenance dose of 120 mg, and lasmiditan is administered at a dose of 100 mg once or twice a day.

In another embodiment the invention provides a method of any one of the embodiments above, wherein galcanezumab is administered in an initial loading dose of 240 mg of followed by a monthly maintenance dose of 120 mg, and lasmiditan is administered at a dose of 200 mg once or twice a day.

In another embodiment the invention provides a method of any one of the embodiments above, wherein galcanezumab is administered at a monthly dose of 120 mg, and lasmiditan is administered at a dose of 50 mg once or twice a day.

In another embodiment the invention provides a method of any one of the embodiments above, wherein galcanezumab is administered at a monthly dose of 120 mg, and lasmiditan is administered at a dose of 100 mg once or twice a day.

In another embodiment the invention provides a method of any one of the embodiments above, wherein galcanezumab is administered at a monthly dose of 120 mg, and lasmiditan is administered at a dose of 200 mg once or twice a day.

In another embodiment the invention provides a method of any one of the embodiments above, wherein galcanezumab is administered at a monthly dose of 120 mg, and lasmiditan is administered at a dose of 50 mg once a day.

In another embodiment the invention provides a method of any one of the embodiments above, wherein galcanezumab is administered at a monthly dose of 120 mg, and lasmiditan is administered at a dose of 100 mg once a day.

In another embodiment the invention provides a method of any one of the embodiments above, wherein galcanezumab is administered at a monthly dose of 120 mg, and lasmiditan is administered at a dose of 200 mg once a day.

Prior treatments of migraine may leave significant numbers of patients without adequate treatment. For instance, up to 40% of migraine attacks, ~30% of patients, fail to respond to a particular triptan, because of suboptimal efficacy or tolerability issues (See Dodick DW. Headache. 2005;45:156-162, and Tepper DE. Headache. 2013(53)577-578). Because of their vasoconstricting effects, these medications may have contraindications, warnings, and precautions for patients with cardiovascular risk factors and disease (See Alwhaibi M, et al. Pain Res Treat. 2016; 2016:8538101, and Gilmore B, Michael M. Am Fam Physician. 2011(83)271-280). In addition, prior therapies often are limited by the onset of migraine overuse headaches, where patients may be limited in the number of treatments they may use in a particular window of time to avoid the onset of migraine overuse headache (See e.g. Diener, H.C. et al., Chronic Headache Due to Overuse of Analgesics and Anti-Migraine Agents. Dtsch Arztebl Int 2018; 115: 365-70). Thus, for prior monotherapy or dual therapy migraine treatments for prior monotherapy or dual therapy migraine treatments, a substantial fraction of patients may fail to achieve headache relief and/or freedom from pain in response to treatment. Further, some patients, referred to herein as therapy resistant migraine patients, will fail to successfully manage their migraine attacks and will suffer from migraines which are refractory to two or more prior monotherapy and/or dual therapy treatment regimens. As defined herein, therapy resistant migraine patients will be those that continue to suffer from 3 or more migraine days per month despite two or more prior monotherapy and/or dual therapy treatment regimens. As used herein, two or more prior monotherapy and/or dual therapy treatment regimens means prior unsatisfactory treatment attempts with a monotherapy or dual therapy regimen, such as triptans, ergotamines, nonsteroidal anti-inflammatory drugs (NSAIDs), nonnarcotic analgesics, blood pressure medications, anticonvulsants, antidepressants, serotonin antagonists, onabotulinum toxin, and caffeine, either alone or two such agents in combination. Further, a population of patients will fail to successfully manage their migraine attacks with either galcanezumab or lasmiditan individually.

These inadequately controlled migraine patients may have a number of migraine day's per-month that continues to be significantly disabling. An unsatisfactory treatment attempt is one in which the patient concludes after a full course of therapy that their symptoms were not alleviated to an extent such that disability was avoided. Disability measures for migraine are well known to the skilled artisan, such as the Migraine Disability Assessment, where a total score ≥11 may represent moderate-to-severe headache-related disability. In embodiments of the present invention a Migraine Disability Assessment of 10 or less, or an equivalent assessment by measures known to the skilled artisan, represents avoidance of disability. Preferably, the combination methods of the present invention provide relief of migraine disability such that patients report a total score on the Migraine Disability Assessment of 10 or less. Preferably, in embodiments of the present invention a Migraine Disability Assessment or an equivalent assessment by measures known to the skilled artisan will demonstrate no clinically disability. Preferably, the combination methods of the present invention provide relief of migraine disability such that post administration of lasmiditan, the migraine patient is free of significant clinical disability wherein the patient does not report complete disability, or needing bed rest, or marked interference with daily activities. More preferably, the combination methods of the present invention provide relief of migraine disability such that post administration of lasmiditan, the migraine patient is free of mild interference. More preferably the combination methods of the present invention provide relief of migraine disability such that post administration of lasmiditan, the migraine patient is not at all disabled. Preferably patients treated by the combination methods provided herein avoid the onset of migraine overuse headache.

Symptomatic relief such as headache pain relief, or relief from the patient's most bothersome symptom, can be for example defined as efficacy according to the clinical study protocols provided herein. Preferably, the combination methods of the present invention provide relief of headache pain, and/or relief from the patients' most bothersome symptom. Headache pain relief as used herein is assessed by reduction in pain severity from moderate or severe at baseline to mild or none, or a reduction in pain severity from mild at baseline to none, at 2 hours postdose. Headache pain free as used herein is a reduction in pain severity from mild, moderate, or severe at baseline to none at the indicated assessment time. The most bothersome symptom (MBS) is identified by participants at the onset of the migraine attack, prior to dosing, from the associated symptoms of nausea, phonophobia, and photophobia. Most bothersome symptom-free as used herein refers to a patient reported outcome of being free of their migraine-associated MBS at 2 hours postdose, the MBS being defined as the associated symptom present and identified as the MBS prior to dosing.

As used herein refractory migraine includes but is not limited to refractory chronic migraine and/or refractory episodic migraine. Means of identification of refractory migraine patients are known to the skilled artisan. For example, refractory chronic migraine is recognized by the skilled artisan, as illustrated in the proposed criteria for this condition provided by the European Headache Federation (EHF) (*See* Headache Classification Committee of the International Headache Society (IHS). The International Classification of Headache Disorders, 3rd edition). The EHF recommends that refractory chronic migraine be defined as ICHD-3 beta chronic migraine without medication overuse in patients who have failed to respond to treatment with at least three preventive medications at adequate dosages, each with trials of at least 3 months. The proposed criteria can be briefly described as follows: A. ICHD-3 beta chronic migraine, with no medication overuse; B. prophylactic migraine medications in adequate dosages used for at least 3 months each; C. contraindications for or no effect of preventive medication with at least three drugs from the following classes: Beta blockers (Propranolol up to 240 mg daily, Metoprolol up to 200 mg daily, Atenolol up to 100 mg daily, Bisoprolol up to 10 mg daily), Anticonvulsants (Valproate acid up to 1.5 g daily, Topiramate up to 200 mg daily), Tricyclics (Amytriptyline up to 150 mg daily), or others (Flunarizine up to 10 mg daily, Candesartan up to 16mg daily, OnabotulinumtoxinA 155-195 U according to the PREEMPT protocol), and D. Adequate treatment of psychiatric or other comorbidities by multidisciplinary team, if available.

The combination treatment methods of the present invention are believed to provide improved migraine treatment, including in patients inadequately controlled by lasmiditan or galcanezumab therapy alone, and/or patients who suffer from therapy resistant migraine wherein the patients migraines have been refractory to two or more prior monotherapy and/or dual therapy treatment regimens, and further provide a particularly advantageous combination of pharmacological benefits, comprising rapid (particularly within two hours, preferably within one hour, and more preferably within 30 minutes post administration of lasmiditan), safe, and effective reduction and/or elimination of headache pain, and at the same time, provide a clinically tolerable level of adverse effects such as dizziness, paresthesia, and somnolence. The combination treatment methods of the present invention may provide these benefits in part by allowing the migraine patient to adequately treat their migraine episodes with a lower dose of lasmiditan, for instance 100 mg, or 50 mg, and more preferably to do so with a single dose per day thereby avoiding the need for a second dose per day. In this respect, the combination treatment methods of the present invention provide migraine patients with significant reduction, and/or more preferably with freedom from significant migraine symptoms and disability. In another respect, the combination treatment methods of the present invention provide migraine patients with significant reduction, and more preferably, with freedom from significant migraine symptoms and disability, for a sustained period of time, for example, 24 hours post administration of lasmiditan, or preferably 48 hours post administration of lasmiditan.

As used herein, "combination therapy" or "in combination" includes the administration of lasmiditan and a CGRP antagonist as part of a specific treatment regimen intended to provide the beneficial effect from the co-action of these therapeutic agents. The beneficial effect of the combination includes, but is not limited to, pharmacokinetic and/or pharmacodynamic co-action resulting from the combination of therapeutic agents. Administration of these therapeutic agents in combination typically is carried out over a defined time of period (usually minutes, hours, days or weeks depending upon the combination selected). Combination therapy is intended to embrace administration of the indicated therapeutic agents in a sequential manner, that is, wherein each therapeutic agent is administered at a different time, as well as administration of these therapeutic agents, in a substantially simultaneous manner. Administration can be accomplished, for example, by administering to the subject a single oral dosage form having a fixed ratio of each therapeutic agent or in multiple, single oral dosage forms for each of the therapeutic agents, or by administering an oral dosage form of lasmiditan and an injectable dosage form of galcanezumab. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intramuscular or intravenous injection, while the other therapeutic agent of the combination may be administered orally. Alternatively, for example where applicable, all therapeutic agents may be administered orally or all therapeutic agents may be administered by intravenous injection. The sequence in which the therapeutic agents are administered is not narrowly critical.

As used herein, "once a day" means lasmiditan is administered one time in a 24 hour period, or one time in a calendar day. As used herein, "once a day" means lasmiditan is administered one time in a 24 hour period, or one time in a calendar day, for the prevention or treatment of a migraine attack. As used herein, "once a day" means lasmiditan is administered one time in a 24 hour period, or one time in a calendar day, for the treatment of a migraine attack, and such treatment may occur for two or more days consecutively.

As used herein "monthly" means galcanezumab is administered one time in a 30 day period, or one time in a calendar month. As used herein "monthly" means galcanezumab is administered one time in a 30 day period, or one time in a calendar month, and the timing of administration in this period may vary. Preferably as used herein "monthly" means galcanezumab is administered one time in a 30 day period, or one time in a calendar month, and is administered on or about the same calendar day each month so as to provide a regular interval of administration.

As described herein lasmiditan is administered in combination with a CGRP antagonist, such as galcanezumab, to abort migraines. In one embodiment, both the lasmiditan and CGRP antagonist are administered for acute migraine relief. In another embodiment, the CGRP antagonist such as galcanezumab is administered for prophylaxis and the lasmiditan is administered for acute migraine relief of breakthrough pain. The present invention also provides a method of treating a condition related to elevated levels of CGRP, preferably headaches and/or migraines comprising administering to a patient in need thereof a therapeutically effective amount of a combination of lasmiditan and a CGRP antagonist such as galcanezumab of the present invention. Some embodiments of the present invention provide a method of treating migraine, episodic headache, chronic headache, chronic cluster headaches, and/or episodic cluster headaches comprising administering to a patient in need thereof a therapeutically effective amount of a combination of lasmiditan and a CGRP antagonist such as galcanezumab.

In those instances where the disorders which can be treated by combinations of the present invention are known by established and accepted classifications, such as migraine, episodic headache, chronic headache, chronic cluster headaches, and/or episodic cluster headaches, their classifications can be found in various sources. For example, at present, the fourth edition of the Diagnostic and Statistical Manual of Mental Disorders (DSM-IV^{™}) (1994, American Psychiatric Association, Washington, D.C.), provides a diagnostic tool for identifying many of the disorders described herein. Also, the International Classification of Diseases, Tenth Revision (ICD-10), provides classifications for many of the disorders described herein. The skilled artisan will recognize that there are alternative nomenclatures, nosologies, and classification systems for disorders described herein, including those as described in the DSM-IV and ICD-10, and that terminology and classification systems evolve with medical scientific progress. Migraine patients can further be diagnosed with migraine, with or without aura (1.1 and 1.2), as defined by International Headache Society (IHS) International Classification of Headache Disorders, 3rd edition, (ICHD-3) beta version (The International Classification of Headache Disorders, 3rd edition (beta version), Cephalalgia 2013; 33: 629-808).

The term "pharmaceutical" or "pharmaceutically acceptable" when used herein as an adjective, means substantially non-toxic and substantially non-deleterious to the recipient. By "pharmaceutical composition" it is further meant that the carrier, solvent, excipients and salt must be compatible with the active ingredient of the composition (e.g. a compound of the invention). It is understood by those of ordinary skill in this art that the terms "pharmaceutical formulation" and "pharmaceutical composition" are generally interchangeable, and they are so used for the purposes of this application.

Additionally, the compounds of the present invention, for example, the salts of the compounds, can exist in either hydrated or unhydrated (the anhydrous) form. Nonlimiting examples of hydrates include monohydrates, dihydrates, etc. When the compounds of this invention are amines, they are basic in nature and accordingly react with any of a number of inorganic and organic acids to form pharmaceutically acceptable acid addition salts. The term "acid addition salt" refers to a salt of a compound prepared by reaction of the compound with a mineral or organic acid. The compounds of the present invention form pharmaceutically acceptable acid addition salts with a wide variety of organic and inorganic acids and include the physiologically acceptable salts which are often used in pharmaceutical chemistry. Such salts are also embodiments of this invention. A "pharmaceutically-acceptable (acid) addition salt" is formed from a pharmaceutically-acceptable acid as is well known in the art. Such salts include the pharmaceutically acceptable salts exemplified in Berge, S.M, Bighley, L.D., and Monkhouse, D.C., J. Pharm. Sci., 66:1, (1977), which are well known to those skilled in the art.

The term "effective amount" means an amount of lasmiditan capable of activating 5-HT-1F receptors or an amount of CGRP antagonist capable of inhibiting the action of CGRP. In a preferred embodiment, "effective amount" means an amount of lasmiditan and an amount of CGRP antagonist capable of rendering a patient pain free at 2 hours post headache treatment with lasmiditan.

The term "treating" or "treatment", as used herein, means to cure an already present disease state or condition, e.g., a migraine or headache in a patient or subject. Treating can also include inhibiting, i.e. arresting the further development of a disease state or condition, and relieving or ameliorating, i.e. causing regression of the disease state or condition, e.g., a migraine. The term "preventing" or "prevention", as used herein means, to completely or almost completely stop a disease state or condition from occurring in a patient or subject, especially when the patient or subject is predisposed to such or at risk of contracting a disease state or condition, e.g., a migraine.

Throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

One skilled in the art of preparing formulations can readily select the proper form and mode of administration depending upon the particular characteristics of the compound selected, the disorder or condition to be treated, the stage of the disorder or condition, and other relevant circumstances (See, e.g., Remington: The Science and Practice of Pharmacy, L.V. Allen, Editor, 22nd Edition, Pharmaceutical Press, 2012). In particular, the components of the present combinations may be combined in the same formulation where appropriate, or alternatively they can be formulated separately.

In a separate formulation, lasmiditan is usually mixed with an excipient, diluted by an excipient or enclosed within such a carrier which can be in the form of a capsule, sachet, paper or other container. The CGRP antagonist is suitably formulated separately. When the excipient serves as a diluent, it can be a solid, semi-solid, or liquid material, which acts as a vehicle, carrier or medium for the active ingredient. Thus, the formulations can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, gels, suppositories, sterile injectable solutions, and sterile packaged powders. Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compounds of the invention can be formulated so as to provide quick, sustained or delayed release of the active ingredient after administration to the patient by employing procedures known in the art.

### Example Clinical Studies

The following clinical study designs further illustrate the invention, but should not be construed to limit the scope of the invention in any way. Below are provided examples of studies of lasmiditan in combination with galcanezumab in the treatment of migraine. It will be understood by the skilled artisan that similar studies can be conducted with patients who have been unable to successfully manage their migraine attacks with either lasmiditan or galcanezumab individually. It will be understood by the skilled artisan that similar studies can be conducted with patients, referred to herein as therapy resistant migraine patients, who have migraine attacks which are refractory to two or more prior monotherapy and/or dual therapy treatment regimens. The skilled artisan can conduct similar studies with patients suffering from a headache selected from the group consisting of episodic headache, chronic headache, chronic cluster headache, or episodic cluster headache, including patients with therapy resistant headaches. The skilled artisan may readily identify, using methods described herein and methods known in the art, patients who have been unable to successfully manage their migraine attacks with either lasmiditan or galcanezumab individually, and/or therapy resistant migraine patients, who have migraine attacks which are refractory to two or more prior monotherapy and/or dual therapy treatment regimens, wherein said patients so identified may be subjects for clinical studies such as those described herein.

Methods of conducting such clinical trials are known to the skilled artisan, and illustrated for instance by the citations for published lasmiditan and galcanezumab clinical studies provided herein. Methods to assess migraine treatments include Patient Reported Outcomes (PRO) such as Quality of Life (QOL) Measures, including for example: Migraine-Specific Quality of Life, version 2.1 (MSQ v2.1), Headache Impact Test-6 (HIT-6), Migraine Disability Assessment Scale (MIDAS), Migraine Specific Quality of Life Questionnaire (MSQoL). In addition, an ePRO daily diary can be used to record headache and other migraine symptoms. Based on the diary data, using an automated algorithm, the days can be categorized as Migraine Headache Days (MHDs) (including probable MHD). A probable migraine is defined as a headache with or without aura and lasting ≥ 30 minutes but missing one of the migraine features in the ICHD-3 beta criteria. The monthly number of MHDs with acute migraine medication use can be obtained through the ePRO diary, and the PGI-S, MSQ, and MIDAS assessments are performed at the study site at every monthly visit for PGI-S and MSQ and on months 3 and 6 for MIDAS with the use of a slate device. A study design can be formulated to compare the efficacy of each combination dosing regimen compared with placebo, or galcanezumab treatment alone, or lasmiditan treatment alone, on the overall mean change from baseline in the number of monthly migraine headache days (MHDs) during the treatment phase, based on the ePRO or other relevant clinical data. Other possible outcome measures can be the mean proportion of patients with reduction from baseline in monthly MIMs during the double-blind treatment phase, the mean change from baseline in the Role Function-Restrictive (R-FR) domain score of the Migraine-Specific Quality of Life Questionnaire version 2.1 (MSQ v2.1), the mean change from baseline in the Patient Global Impression of Severity (PGI-S) rating (average of selected months), and/or an outcome measure for the Migraine Disability Assessment (MIDAS) total score. These and other migraine treatment assessments are well known to the skilled artisan.

### Acute Treatment of Migraine with Lasmiditan with and without a CGRP antagonist such as galcanezumab by Intravenous Administration

To demonstrate the efficacy of the combination of lasmiditan and galcanezumab, the following study is conducted once as written below, and once wherein the patients under evaluation have been administered an initial loading dose of 240 mg of galcanezumab followed by a monthly maintenance dose of 120 mg of galcanezumab. Treatment arms of the studies may include unit doses of 50 mg, 100 mg, or 200 mg of lasmiditan for oral administration.

In a multi-centre, placebo-controlled, double-blind, group-sequential, adaptive treatment-assignment, proof-of-concept and dose-finding study, 130 patients are treated in-hospital during a migraine attack. Patients are allocated to an intravenous dose level of lasmiditan or placebo in small cohorts. The starting dose is 2.5 mg. Subsequent doses are adjusted, up or down, according to the safety and efficacy seen in the preceding cohort. The primary outcome measure is headache response defined as improvement from moderate or severe headache at baseline to mild or no headache at 2 hours post-dose. The study is designed to explore the overall dose response relationship but is not powered to differentiate individual doses from placebo, nor to detect effect differences for other migraine symptoms.

Forty two patients receive placebo and 88 receive lasmiditan in doses of 2.5 to 45 mg. Patients are observed in the clinic for 4 hours after treatment and use a diary card to record symptoms and adverse events for up to 24 hours. The study is terminated when a dose meets predefined efficacy stopping rules. Patients treated in the 10, 20, 30 and 45 mg lasmiditan dose groups are evaluated for a 2h headache response, compared to the placebo group. Patient global impression at 2h and lack of need for rescue medication are also evaluated for statistically significant linear correlations with dose. At intravenous lasmiditan doses of 2.5 to 45 mg, the effectiveness of lasmiditan in combination with concurrent galcanezumab treatment is evaluated for the acute treatment of migraine.

### Methods

The study can be conducted at multiple sites and in accordance with the Declaration of Helsinki and internationally accepted standards of Good Clinical Practice. Prior to initiation it requires approval by the relevant regulatory authorities and independent ethics committees. All subjects are limited to those providing written informed consent.

### Study Design

The study uses a prospective, randomized, double-blind, placebo-controlled design with group-sequential adaptive-treatment assignment (Olesen J et al., N Engl J Med 2004: 350: 1104-10; Hall DB et al., Contemporary Clinical Trials 2005; 26: 349-63). Patients are allocated to a dose level of lasmiditan in small cohorts, with the first 20 cohorts consisting of 6 patients (4 receive lasmiditan and 2 placebo) and subsequent cohorts of 5 patients (4 lasmiditan and 1 placebo). The first cohort is allocated to the 2.5 mg dose level. The dose used in subsequent cohorts depends on the headache response (moderate or severe headache reduced to mild or none at 2 hours) of the previous cohort: if 2 or less of the 4 active-treated patients have responded, the dose is increased, and if 3 or more of the 4 active-treated patients have responded, the dose is reduced. The dose adjustment rules are chosen to identify doses of lasmiditan with efficacy similar to or better than an oral triptan. This dose escalation or reduction sequence will be modified if 2 or more active-treated patients in any cohort experience a severe non-serious adverse event, in which case the dose will be reduced for the next cohort irrespective of the response rate. The occurrence of a drug-related serious adverse event will lead to automatic suspension of the randomization pending a safety review. The lowest permissible dose of lasmiditan is 1 mg and the highest is 60 mg.

The up-and-down dose adjustment process is terminated with the selection of an effective dose when the following criteria have been met: at least 5 blocks of patients have been treated at this dose, and for at least 4 blocks the decision rule called for a dose decrease. Alternatively, the dose selection process could be terminated, without the selection of an effective dose, if 5 consecutive blocks of patients have been treated at the top dose with the escalation rules calling for a dose increase each time.

### Patient Screening and Selection

Patients are initially screened for eligibility at an out-patient visit outside a migraine attack, and are invited to return to the clinic for treatment with study medication of a new, moderate or severe migraine attack within 4 hours of onset. On return to the clinic, eligibility for the study is reconfirmed and the patient is randomized. Patients are eligible for the study if they are between 18 and 65 years of age and have at least a 1 year history of migraine with or without aura fulfilling the IHS diagnostic criteria 1.1 and 1.2.1 (2004), with a migraine onset before the age of 50 years (Headache Classification Subcommittee of the International Headache Society. The International Classification of Headache Disorders (second edition). Cephalalgia 2004: 24; Suppl1:1-160). Patients have to be experiencing between 1 and 8 migraine attacks a month and not be using migraine prophylactic medication. Patients are in good general health and have no evidence of vascular disease or hypertension. Patients with previous intolerance of triptans are excluded. Pregnant or breast-feeding women are excluded, as are women of childbearing potential who are not using a highly reliable form of contraception.

### Study Procedures

On return of the patient to the clinic, instructions for dilution of study drug are obtained from an online randomization system by a pharmacist or other study personnel, independent of the investigator, and the study drug for infusion is prepared. Both investigator and pharmacist are blinded with regard to active or placebo and only the pharmacist knows the dilution. All patients receive a 60 ml intravenous infusion over 20 minutes. Efficacy and safety data before and after administration of study drug are entered immediately into an electronic data capture system, allowing the headache response to be used to drive dose-allocation for subsequent cohorts.

After baseline assessments are completed, lasmiditan or placebo is infused intravenously over 20 minutes and the patient is monitored for safety and efficacy for at least 4 hours. Data are entered concurrently into an online electronic data capture system. Patients are discharged from the clinic after 4h and continued to record migraine symptoms and adverse events until 24h using a diary card.

### Symptom Evaluation

A number of different symptoms are evaluated. The severity of headache is measured on a four point scale with 0=no pain, 1=mild pain, 2=moderate pain, 3=severe pain. Associated symptoms (nausea, vomiting, photophobia, phonophobia) are recorded as present or absent. Disability is documented on a four point scale with 0=no disability, 1=mild disability, 2=moderate disability, 3=severe disability. Data for the patient global impression is collected on a seven point scale with 1=very much better, 2=much better, 3=a little better, 4=no change, 5=a little worse, 6=much worse, 7=very much worse.

The primary efficacy measure is headache response, defined as a reduction in headache severity from moderate or severe at baseline to mild or no headache at 2 hours after initiation of infusion of study drug (HIS Clinical Trials Subcommittee. Guidelines for Controlled Trials in Migraine: second edition, Cephalalgia 2000: 20: 765-786). The secondary efficacy measures are: rates of headache response at 10 min, 20 min, 40 min, 60 min, 90 min, 180 min, and 240 min after initiation of study drug infusion; rates of headache free (reduction from moderate or severe headache at baseline to no headache pain) at 10 min, 20 min, 40 min, 60 min, 90 min, 120 min, 180 min, and 240 min after initiation of study drug; rates of sustained response, defined as a moderate or severe headache at baseline which became mild or no headache at 2h after initiation of study drug and which did not recur (become moderate or severe) within 24h of initiation of study drug; rates of sustained pain-free, defined as a moderate or severe headache at baseline which became no headache at 2h after initiation of study drug and which did not recur (become mild, moderate or severe) within 24h of initiation of study drug; presence of nausea, vomiting, photophobia and phonophobia, and degree of clinical disability throughout the study course; proportion of patients using rescue medication between 2 and 24h after initiation of study drug, and patient global impression 2h after initiation of study drug.

### Statistical Methods

The target sample size of at most 160 patients, with at least 20 patients treated with an effective dose level and at least 10 patients treated with placebo, is selected to provide appropriate preliminary data on which to choose a dose range for further evaluation. The statistical properties of the hypothesis tests to compare one or more dose levels to placebo when doses are allocated using the group sequential adaptive treatment assignment design are not known. Formal statistical tests are therefore not used to declare the study to be "positive" or "negative" and the study is not powered for statistical significance. Furthermore, the sample size is not powered for statistical considerations.

At the conclusion of the study, the headache response rates are summarized by dose level. The Mantel-Haenszel test is used to test for a dose-response relationship. Fisher's exact test is used to compare the headache response rates for the selected dose versus placebo. In all analyses, the results for each dose level (including placebo) are combined across all blocks where that dose was used. All patients who receive any study medication are included in the analysis population. The patients are analyzed according to the treatment and dose level they actually receive.

### Efficacy

The linear association between response rate and dose level is statistically assessed with the Mantel-Haenszel test for trend. The proportion of patients in each group who achieve a headache response at time points from 10 min to 4h are tabulated. The main secondary efficacy parameters are tabulated for each group including patient global impression at 2h and use of rescue medication up to 24h. Secondary Efficacy Parameters are: Pain freedom at 2 hrs, Sustained pain response, Sustained pain free, Nausea at 2 hrs, Photophobia at 2 hrs, Phonophobia at 2 hrs, No/mild disability at 2 hrs, Use of rescue medication, 2-24 hrs, Impression: very much/much better at 2hrs.

The acute antimigraine efficacy of lasmiditan with or without concurrent galcanezumab treatment is tested. An up-and-down dose-adaptive study design is used to minimize patient exposure to study drug or placebo while still rapidly and reliably screening for efficacy and tolerability across a wide dose range. The onset of headache relief may be evident at 20 to 40 min. after the start of a 20 min. intravenous infusion.

The clinician can evaluate the study efficacy outcomes to determine the percent reporting disability following intravenous administration of lasmiditan, the percent of moderate or severe disability reported, the patient global impressions, and the percent of patients who report feeling "very much" or "much better" 2 hours post dose. The clinician can also evaluate secondary endpoints (photophobia, phonophobia, and nausea).

### A Double Blind Randomized Placebo-Controlled Parallel Group Dose-Ranging Study of Oral Lasmiditan with and without Galcanezumab in the Acute Treatment of Migraine

To demonstrate the efficacy of the combination of lasmiditan and galcanezumab, the following study is conducted once as written below, and once wherein the patients under evaluation have been administered an initial loading dose of 240 mg of galcanezumab followed by a monthly maintenance dose of 120 mg of galcanezumab.

A study is conducted to evaluate the efficacy (headache response at two hours) of a range of oral doses of Lasmiditan. A secondary objective is to explore the time course and effect of a range of dose levels of lasmiditan on features of the migraine including: headache response, proportion of patients pain-free, headache recurrence, nausea, photophobia, phonophobia, vomiting, disability, use of rescue medication and patient global impression. The study explores the safety and tolerability of a range of doses of lasmiditan in terms of adverse events, physical exam, vital signs, laboratory evaluations, and ECGs. The study protocol is outlined below.

This is a prospective randomized, double-blind, placebo-controlled dose-ranging study in subjects with migraine. Patients are asked to treat a single migraine attack with study medication at home. Each subject's study participation consists of a screening visit with a telephone contact within 5 days to confirm eligibility, a treatment period of up to 8 weeks during which the subject is asked to treat one migraine attack with a single dose of one of four dose levels of oral

### lasmiditan or placebo, and a follow-up visit within 14 days of treating an attack.

Following screening, subjects are randomly assigned to receive oral lasmiditan (50, 100, 200 or 400 mg) or matching placebo to use as the first treatment of a new migraine attack. Subjects are instructed not to treat an attack until their eligibility has been confirmed by phone once all screening evaluations are complete. Once eligibility is confirmed subjects are asked to treat their next migraine attack within 4 hours of its onset providing that the headache severity is at least moderate at that time and not improving. Subjects record their response over the next 48 hours using a diary card. Subjects are asked not to use rescue medication until at least 2 hours after taking the study medication. Once an attack has been treated, subjects contact the clinic to schedule a follow-up visit as soon as possible and within 14 days of treatment. Patients are allocated to one of four dose levels of lasmiditan or matching placebo in the ratio 1:1:1:1:1 according to a predefined randomization list. At least 340 patients treat one attack with study medication.

### Criteria for Inclusion/Exclusion:

Inclusion: Subjects are included in the study only if all the following criteria are met: Patients with migraine with or without aura fulfilling the IHS diagnostic criteria 1.1 and 1.2.1(2004); History of migraine for at least 1 year; Migraine onset before the age of 50 years; History of 1 - 8 migraine attacks per month; Male or female patients aged 18 to 65 years; Female patients of child-bearing potential must be using a highly effective form of contraception (e.g., combined oral contraceptive, IUD, abstinence, vasectomized partner); Able and willing to give written informed consent; Able and willing to complete a migraine diary card to record details of the attack treated with study medication.

Exclusion: Subjects are excluded from the study if any of the following criteria are met: History of life threatening or intolerable adverse reaction to any triptan; Use of prescription migraine prophylactic drugs within 30 days prior to Screening Visit and during study participation (other than galcanezunab as by design); Pregnant or breast-feeding women; Women of child-bearing potential not using highly effective contraception; History or evidence of coronary artery disease, ischemic or hemorrhagic stroke, epilepsy or any other condition placing the patient at increased risk of seizures; History of hypertension (controlled or uncontrolled); History of orthostatic hypotension; Sitting BP >160mmHg systolic or >90mmHg diastolic on 2 repeated measurements at screening; Current use of hemodynamically active cardiovascular drugs; History within the previous 3 years or current evidence of abuse of any drug, prescription or illicit, or alcohol; Significant renal or hepatic impairment; Previous participation in this clinical trial; Participation in any clinical trial of an experimental drug or device in the previous 30 days; Any medical condition or laboratory test which in the judgment of the investigator makes the patient unsuitable for the study; Known Hepatitis B or C or HIV infection; Subjects who are employees of the sponsor; Relatives of, or staff directly reporting to, the investigator; Patients with known hypersensitivity to Compound I, other 5-HT_{1F} receptor agonists or to any excipient of Compound drug product; Patients who were treated with study medication in a previous lasmiditan study (Patients screened but not treated under that protocol are not excluded).

### Criteria for Evaluation include:

Efficacy/Pharmacodynamics: Headache severity (4 point scale: none, mild, moderate, severe); Headache recurrence within 48 hours; Presence or absence of nausea; phonophobia, photophobia, vomiting; Disability (4 point scale: none, mild, moderate, severe); Requirement for rescue medication between 2 and 48 hours (yes or no); Patient global impression (7 point scale); Time to headache relief and time to pain free.

### Safety:

Physical examination; Adverse events (spontaneously reported); Vital signs; 12-lead electrocardiograms; Clinical laboratory parameters; Statistical Analysis.

### Efficacy:

This multi-center, randomized, double-blind, parallel-group, placebo-controlled clinical study is designed to evaluate the efficacy and safety of oral lasmiditan with and without concurrent galcanazumab in the acute treatment of migraine. The proportion of subjects with headache relief 2 hours post dose is the primary efficacy parameter. The primary efficacy analysis tests the null hypothesis that the proportions of subjects with headache relief 2 hours post dose are the same in the five study arms, versus the alternative hypothesis of a positive linear trend in the response rates, using the Cochran-Armitage test for trend. The primary analysis is performed in the modified intent-to-treat population, defined as all subjects who treat an attack with study medication, using a one-sided test at the 5% level of significance. Patients who fail to document headache severity at 2 hours or use of rescue medication before that time point are excluded from the analysis set.

Using a logistic regression model including the data from all five treatment groups, additional efficacy analyses compare each active dose group to the placebo group. Additional analyses are also be based on a per-protocol set of subjects.

The sample size is estimated assuming a response rate of 40% in the placebo arm and a 65% rate in the highest active dose arm. Assuming that the treatment groups are equally spaced and that the response odds ratios are equal between pairs of adjacent dose groups, the required sample size is estimated using the approach of Nam (1987). Based on 1:1:1:1:1 randomization, a total sample size of 330 patients (66 per group) is required for 90% power, based on a one-sided test at the 5% level of significance.

Safety: Adverse events can be summarized, and event rates can be presented by treatment group. Laboratory data is summarized by treatment group in terms of change from baseline status. A safety population may consist of all randomized patients who received at least one dose of study drug or placebo. Adverse events can be coded by Medical Dictionary for Regulatory Activities (version 19.1). Safety parameters may be calculated as the treatment-emergent adverse events (TEAEs), serious adverse events (SAEs), deaths, discontinuations due to adverse events, discontinuation rates, vital signs, body weight, and immunogenicity.

A primary analysis can evaluate the efficacy of each combination dosing regimen compared with placebo, or galcanezumab alone, or lasmiditan alone, on the overall mean change from baseline in the number of monthly migraine headache days (MHDs) during the treatment phase, based on the ePRO or other relevant clinical data. Other outcome measures can be the mean proportion of patients with reduction from baseline of ≥ 50%, ≥ 75%, and 100% in monthly MHDs during the double-blind treatment phase. The mean change from baseline in the Role Function-Restrictive (R-FR) domain score of the Migraine-Specific Quality of Life Questionnaire version 2.1 (MSQ v2.1) can be calculated, as an average of selected months of the study. The overall mean change from baseline in the number of MHDs during the double-blind treatment phase can be calculated. A mean change from baseline in the Patient Global Impression of Severity (PGI-S) rating (average of selected months) can be calculated. An outcome measure for the Migraine Disability Assessment (MIDAS) total score can be calculated at a selected timepoint, such as the end of the study.

Monotherapy phase III studies for both lasmiditan and galcenazeumab, used separately, have been conducted and published. See for instance for lasmiditan, *Phase 3 Studies (SAMURAI, SPARTAN) of Lasmiditan Compared to Placebo for Acute Treatment of Migraine* (S50.008), Linda A. Wietecha, Bernice Kuca, Josephine Asafu-Adjei, Sheena K. Aurora, Neurology April 2018, 90 (15 Supplement) S50.008; where the authors have reported that at 2 hours post-first dose, significantly greater proportions of patients (p<0.001) were headache pain-free (lasmiditan 200 mg: SAMURAI 32.2%, SPARTAN 38.8%; placebo: SAMURAI 15.3%, SPARTAN 21.3%) and most bothersome symptom (MBS)-free (lasmiditan 200 mg: SAMURAI 40.7%, SPARTAN 48.7%; placebo: SAMURAI 29.5%, SPARTAN 33.5%) with lasmiditan 200 mg compared with placebo. For both endpoints, significance was also noted for other lasmiditan dose groups (100 mg, 50 mg) compared to placebo. The most frequently reported TEAEs with lasmiditan (≥ 2% and greater than placebo) after the first dose were dizziness, paresthesia, somnolence, fatigue, nausea, and lethargy, and most events were mild-to-moderate in severity. From this analysis, the authors concluded the primary and key secondary endpoints were met and safety outcomes were consistent across the two Phase 3 studies. And see for instance for galcanezumab, Efficacy and safety of galcanezumab for the prevention of episodic migraine: Results of the EVOLVE-2 Phase 3 randomized controlled clinical trial, Vladimir Skljarevski, Manjit Matharu, Brian A Millen, Michael H Ossipov, Byung-Kun Kim and Jyun Yan Yang, Cephalalgia 0(0) 1-13, 2018, where the authors concluded mean monthly migraine headache days were reduced by 4.3 and 4.2 days by galcanezumab 120 and 240 mg, respectively, and 2.3 days by placebo. The group differences (95% CIs) versus placebo were 2.0 (-2.6, -1.5) and 1.9 (-2.4, -1.4), respectively. Both doses were superior to placebo for all key secondary endpoints. Injection site pain was the most common treatment-emergent adverse event, reported at similar rates in all treatment groups. Both galcanezumab doses had significantly more injection site reactions and injection site pruritus, and the 240 mg group had significantly more injection site erythema versus placebo. From this analysis the authors concluded galcanezumab 120 or 240 mg given once monthly was efficacious, safe, and well tolerated.

It is believed that the combination of lasmiditan and galcanezumab for use in the treatment of migraine will be superior to either monotherapy alone, particularly in certain previously unsuccessfully treated populations, by combined action on the CGRP pathway in combination with a complimentary action of lasmiditan to decrease glutamate signaling. It is believed the combination of these pharmacological properties will result in superior efficacy for migraine treatment in patients who suffer from therapy resistant migraines. While each agent, namely lasmiditan and galcanezumab alone, has demonstrated efficacy in treatment of migraine, the present invention, which provides a method of treating migraine in a patient, comprising administering simultaneously, separately, or sequentially to a patient in need of such a treatment, an effective amount of lasmiditan in combination with an effective amount of galcanezumab, may provide additional potential advantages for migraine patients, and more particularly for migraine patients who individually do not experience adequate migraine treatment efficacy when treated with either galcanezumab or lasmiditan alone. Thus, potential efficacy provided by the present combination use of galcanezumab and lasmiditan, for treating patients inadequately controlled by lasmiditan or galcanezumab therapy alone, and/or treating migraine patients whose disease has been refractory to two or more prior monotherapy and/or dual therapy treatment regimens, would represent an important additional advancement in migraine therapy. Preferably, the presently provided combination methods of treating migraine in a patient, comprising administering simultaneously, separately, or sequentially to a patient in need of such a treatment, an effective amount of lasmiditan in combination with an effective amount of galcanezumab, may further provide efficacy to these inadequately treated migraine patients such that they may be pain free at 2 hours post treatment with lasmiditan, or more preferably at 1 hour post treatment with lasmiditan, and even more preferably would also experience relief from their most bothersome symptoms at 2 hours post treatment with lasmiditan. Preferably, the patients treated by the combinations of the present invention may potentially also experience sustained pain relief, and/or more preferably freedom from migraine pain, and/or freedom from migraine disability as assessed by methods well known to the skilled artisan, such as the MIDAS assessment or by well-known quality of life measures. Preferably patients treated with the combinations of the present invention would experience three or less migraine days per month, and more preferably not more than one migraine day per month. Preferably, the combinations of the present invention may provide additional potential advantages in the form of efficacy, such as described immediately above, in the treatment of a headache selected from the group consisting of episodic headache, chronic headache, chronic cluster headache, or episodic cluster headache. Preferably, the combination therapies of the present invention will provide improved migraine treatment as described herein, while at the same time demonstrating desirable clinical safety and tolerability.

### List of Embodiments

1. Lasmiditan for use in simultaneous, separate or sequential combination with rimegepant in the oral treatment of migraine in a patient.
2. Lasmiditan for use in simultaneous, separate or sequential combination with ubrogepant in the oral treatment of migraine in a patient.
3. Lasmiditan for use in simultaneous, separate or sequential combination with olcegepant in the oral treatment of migraine in a patient.
4. Lasmiditan for use in simultaneous, separate or sequential combination with MK-8031 in the oral treatment of migraine in a patient.
5. Lasmiditan for use in simultaneous, separate, or sequential combination with rimegepant in the treatment of migraine, episodic headache, chronic headache, chronic cluster headache, or episodic cluster headache in a patient, wherein the patient is suffering from therapy resistant migraine or headache wherein the patient's migraines or headaches have been refractory to two or more prior monotherapy and/or dual therapy treatment regimens.
6. Lasmiditan for use in simultaneous, separate, or sequential combination with ubrogepant in the treatment of migraine, episodic headache, chronic headache, chronic cluster headache, or episodic cluster headache in a patient, wherein the patient is suffering from therapy resistant migraine or headache wherein the patient's migraines or headaches have been refractory to two or more prior monotherapy and/or dual therapy treatment regimens.
7. Lasmiditan for use in simultaneous, separate, or sequential combination with olcegepant in the treatment of migraine, episodic headache, chronic headache, chronic cluster headache, or episodic cluster headache in a patient, wherein the patient is suffering from therapy resistant migraine or headache wherein the patient's migraines or headaches have been refractory to two or more prior monotherapy and/or dual therapy treatment regimens.
8. Lasmiditan for use in simultaneous, separate, or sequential combination with MK-8031 in the treatment of migraine, episodic headache, chronic headache, chronic cluster headache, or episodic cluster headache in a patient, wherein the patient is suffering from therapy resistant migraine or headache wherein the patient's migraines or headaches have been refractory to two or more prior monotherapy and/or dual therapy treatment regimens.
9. A pharmaceutical composition comprising a combination of lasmiditan and rimegepant for use in the oral treatment of migraine in a patient.
10. A pharmaceutical composition comprising a combination of lasmiditan and ubrogepant for use in the oral treatment of migraine in a patient.
11. A pharmaceutical composition comprising a combination of lasmiditan and olcegepant for use in the oral treatment of migraine in a patient.
12. A pharmaceutical composition comprising a combination of lasmiditan and MK-8031 for use in the oral treatment of migraine in a patient.
13. A method for use of lasmiditan in simultaneous, separate, or sequential combination with a calcitonin gene-related peptide (CGRP) antagonist in the treatment of migraine in a patient.
14. A method for use of lasmiditan in simultaneous, separate, or sequential combination with galcanezumab in the treatment of migraine in a patient.
15. A method for use of lasmiditan in simultaneous, separate, or sequential combination with a calcitonin gene-related peptide (CGRP) antagonist in the treatment of migraine in a patient inadequately controlled by lasmiditan or a CGRP antagonist therapy alone.
16. A method for use of lasmiditan in simultaneous, separate, or sequential combination with galcanezumab in the treatment of migraine in a patient inadequately controlled by lasmiditan or galcanezumab therapy alone.
17. A method for use of lasmiditan in simultaneous, separate, or sequential combination with a calcitonin gene-related peptide (CGRP) antagonist in the treatment of migraine in a patient suffering from therapy resistant migraine wherein the patients migraines have been refractory to two or more prior monotherapy and/or dual therapy treatment regimens.
18. A method for use of lasmiditan in simultaneous, separate, or sequential combination with galcanezumab in the treatment of migraine in a patient suffering from therapy resistant migraine wherein the patients migraines have been refractory to two or more prior monotherapy and/or dual therapy treatment regimens.
19. A method of any one of embodiments 10, 12 or 14, wherein galcanezumab is administered in an initial loading dose of 240 mg of followed by a monthly maintenance dose of 120 mg, and lasmiditan is administered at a dose of 50 mg once or twice a day.
20. A method of any one of embodiments 10, 12 or 14, wherein galcanezumab is administered in an initial loading dose of 240 mg of followed by a monthly maintenance dose of 120 mg, and lasmiditan is administered at a dose of 100 mg once or twice a day.
21. A method of any one of embodiments 10, 12 or 14, wherein galcanezumab is administered in an initial loading dose of 240 mg of followed by a monthly maintenance dose of 120 mg, and lasmiditan is administered at a dose of 200 mg once or twice a day.
22. A method of any one of embodiments 10, 12 or 14, wherein galcanezumab is administered at a monthly dose of 120 mg, and lasmiditan is administered at a dose of 50 mg once or twice a day.
23. A method of any one of embodiments 10, 12 or 14, wherein galcanezumab is administered at a monthly dose of 120 mg, and lasmiditan is administered at a dose of 100 mg once or twice a day.
24. A method of any one of embodiments 10, 12 or 14, wherein galcanezumab is administered at a monthly dose of 120 mg, and lasmiditan is administered at a dose of 200 mg once or twice a day.
25. A method of any one of embodiments 10, 12 or 14, wherein galcanezumab is administered at a monthly dose of 120 mg, and lasmiditan is administered at a dose of 50 mg once a day.
26. A method of any one of embodiments 10, 12 or 14, wherein galcanezumab is administered at a monthly dose of 120 mg, and lasmiditan is administered at a dose of 100 mg once a day.
27. A method of any one of embodiments 10, 12 or 14, wherein galcanezumab is administered at a monthly dose of 120 mg, and lasmiditan is administered at a dose of 200 mg once a day.
28. A method for use of lasmiditan in simultaneous, separate, or sequential combination with a calcitonin gene-related peptide (CGRP) antagonist in the treatment of a headache selected from the group consisting of episodic headache, chronic headache, chronic cluster headache, or episodic cluster headache in a patient.
29. A method for use of lasmiditan in simultaneous, separate, or sequential combination with galcanezumab in the treatment of a headache selected from the group consisting of episodic headache, chronic headache, chronic cluster headache, or episodic cluster headache in a patient.
30. A method for use of lasmiditan in simultaneous, separate, or sequential combination with a calcitonin gene-related peptide (CGRP) antagonist in the treatment of a headache selected from the group consisting of episodic headache, chronic headache, chronic cluster headache, or episodic cluster headache in a patient inadequately controlled by lasmiditan or a CGRP antagonist therapy alone.
31. A method for use of lasmiditan in simultaneous, separate, or sequential combination with galcanezumab in the treatment of a headache selected from the group consisting of episodic headache, chronic headache, chronic cluster headache, or episodic cluster headache in a patient inadequately controlled by lasmiditan or galcanezumab therapy alone.
32. A method for use of lasmiditan in simultaneous, separate, or sequential combination with a calcitonin gene-related peptide (CGRP) antagonist in the treatment of a headache selected from the group consisting of episodic headache, chronic headache, chronic cluster headache, or episodic cluster headache in a patient suffering from therapy resistant headaches wherein the patients headaches have been refractory to two or more prior monotherapy and/or dual therapy treatment regimens.
33. A method for use of lasmiditan in simultaneous, separate, or sequential combination with galcanezumab in the treatment of a headache selected from the group consisting of episodic headache, chronic headache, chronic cluster headache, or episodic cluster headache in a patient suffering from therapy resistant headaches wherein the patients headaches have been refractory to two or more prior monotherapy and/or dual therapy treatment regimens.
34. A method for use of lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered in an initial loading dose of 240 mg of followed by a monthly maintenance dose of 120 mg, and lasmiditan is administered at a dose of 50 mg once or twice a day.
35. A method for use of lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered in an initial loading dose of 240 mg of followed by a monthly maintenance dose of 120 mg, and lasmiditan is administered at a dose of 100 mg once or twice a day.
36. A method for use of lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered in an initial loading dose of 240 mg of followed by a monthly maintenance dose of 120 mg, and lasmiditan is administered at a dose of 200 mg once or twice a day.
37. A method for use of lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 50 mg once or twice a day.
38. A method for use of lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 100 mg once or twice a day.
39. A method for use of lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 200 mg once or twice a day.
40. A method for use of lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 50 mg once a day.
41. A method for use of lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 100 mg once a day.
42. A method for use of lasmiditan in simultaneous, separate, or sequential combination with galcanezumab in the treatment of a headache selected from the group consisting of episodic headache, chronic headache, chronic cluster headache, or episodic cluster headache in a patient, wherein galcanezumab is administered at a monthly dose of 120 mg, and lasmiditan is administered at a dose of 200 mg once a day.
43. A method of treating migraine in a patient, comprising administering simultaneously, separately, or sequentially to a patient in need of such a treatment, an effective amount of lasmiditan in combination with an effective amount of a calcitonin gene-related peptide (CGRP) antagonist.
44. A method of treating migraine in a patient, comprising administering simultaneously, separately, or sequentially to a patient in need of such a treatment, an effective amount of lasmiditan in combination with an effective amount of galcanezumab.
45. A method of treating migraine in a patient, comprising administering simultaneously, separately, or sequentially to a patient in need of such a treatment, an effective amount of lasmiditan in combination with an effective amount of a calcitonin gene-related peptide (CGRP) antagonist, wherein migraine in the patient was inadequately controlled by lasmiditan or a CGRP antagonist therapy alone.
46. A method of treating migraine in a patient, comprising administering simultaneously, separately, or sequentially to a patient in need of such a treatment, an effective amount of lasmiditan in combination with an effective amount of galcanezumab, wherein migraine in the patient was inadequately controlled by lasmiditan or galcanezumab therapy alone.
47. A method of treating migraine in a patient, comprising administering simultaneously, separately, or sequentially to a patient in need of such a treatment, an effective amount of lasmiditan in combination with an effective amount of a calcitonin gene-related peptide (CGRP) antagonist, wherein the patient suffers from therapy resistant migraine wherein the patients migraines have been refractory to two or more prior monotherapy and/or dual therapy treatment regimens.
48. A method of treating migraine in a patient, comprising administering simultaneously, separately, or sequentially to a patient in need of such a treatment, an effective amount of lasmiditan in combination with an effective amount of galcanezumab, wherein the patient suffers from therapy resistant migraine wherein the patients migraines have been refractory to two or more prior monotherapy and/or dual therapy treatment regimens.
49. The method of any one of embodiments 40, 42 or 44, wherein galcanezumab is administered in an initial loading dose of 240 mg of followed by a monthly maintenance dose of 120 mg, and lasmiditan is administered at a dose of 50 mg once or twice a day.
50. The method of any one of embodiments 40, 42 or 44, wherein galcanezumab is administered in an initial loading dose of 240 mg of followed by a monthly maintenance dose of 120 mg, and lasmiditan is administered at a dose of 100 mg once or twice a day.
51. The method of any one of embodiments 40, 42 or 44, wherein galcanezumab is administered in an initial loading dose of 240 mg of followed by a monthly maintenance dose of 120 mg, and lasmiditan is administered at a dose of 200 mg once or twice a day.
52. The method of any one of embodiments 40, 42 or 44, wherein galcanezumab is administered at a monthly dose of 120 mg, and lasmiditan is administered at a dose of 50 mg once or twice a day.
53. The method of any one of embodiments 40, 42 or 44, wherein galcanezumab is administered at a monthly dose of 120 mg, and lasmiditan is administered at a dose of 100 mg once or twice a day.
54. The method of any one of embodiments 40, 42 or 44, wherein galcanezumab is administered at a monthly dose of 120 mg, and lasmiditan is administered at a dose of 200 mg once or twice a day.
55. The method of any one of embodiments 40, 42 or 44, wherein galcanezumab is administered at a monthly dose of 120 mg, and lasmiditan is administered at a dose of 50 mg once a day.
56. The method of any one of embodiments 40, 42 or 44, wherein galcanezumab is administered at a monthly dose of 120 mg, and lasmiditan is administered at a dose of 100 mg once a day.
57. The method of any one of embodiments 40, 42 or 44, wherein galcanezumab is administered at a monthly dose of 120 mg, and lasmiditan is administered at a dose of 200 mg once a day.

## Claims

1. Lasmiditan for use in simultaneous, separate or sequential combination with rimegepant in the oral treatment of migraine in a patient.

2. Lasmiditan for use in simultaneous, separate or sequential combination with ubrogepant in the oral treatment of migraine in a patient.

3. Lasmiditan for use in simultaneous, separate or sequential combination with olcegepant in the oral treatment of migraine in a patient.

4. Lasmiditan for use in simultaneous, separate or sequential combination with MK-8031 in the oral treatment of migraine in a patient.

5. Lasmiditan for use in simultaneous, separate, or sequential combination with rimegepant in the treatment of migraine, episodic headache, chronic headache, chronic cluster headache, or episodic cluster headache in a patient, wherein the patient is suffering from therapy resistant migraine or headache wherein the patient's migraines or headaches have been refractory to two or more prior monotherapy and/or dual therapy treatment regimens.

6. Lasmiditan for use in simultaneous, separate, or sequential combination with ubrogepant in the treatment of migraine, episodic headache, chronic headache, chronic cluster headache, or episodic cluster headache in a patient, wherein the patient is suffering from therapy resistant migraine or headache wherein the patient's migraines or headaches have been refractory to two or more prior monotherapy and/or dual therapy treatment regimens.

7. Lasmiditan for use in simultaneous, separate, or sequential combination with olcegepant in the treatment of migraine, episodic headache, chronic headache, chronic cluster headache, or episodic cluster headache in a patient, wherein the patient is suffering from therapy resistant migraine or headache wherein the patient's migraines or headaches have been refractory to two or more prior monotherapy and/or dual therapy treatment regimens.

8. Lasmiditan for use in simultaneous, separate, or sequential combination with MK-8031 in the treatment of migraine, episodic headache, chronic headache, chronic cluster headache, or episodic cluster headache in a patient, wherein the patient is suffering from therapy resistant migraine or headache wherein the patient's migraines or headaches have been refractory to two or more prior monotherapy and/or dual therapy treatment regimens.
